# EUROPÄISCHE PATENTANMELDUNG

(11) **EP 1 834 660 A1**
(43) Veröffentlichungstag der Anmeldung: **19.09.2007**
(21) Anmeldenummer: 06111351.0
(22) Anmeldetag: 17.03.2006
(51) Int. Cl.: A61M 16/06

(54) **Vorrichtung zum Zuführen von Atmungsgas direkt in die Nase eines Benutzers**

(71) Anmelder: Innosuisse Management AG, 3280 Murten (CH)
(72) Erfinder: Mutti, Franco, 2605, Sonceboz-Sombeval (CH); Gfeller, Jürg, 2613, Villeret (CH)
(74) Vertreter: Scheuzger, Beat Otto

(57) **Zusammenfassung**

Die vorliegende Erfindung betrifft eine Vorrichtung zum Zuführen von Atmungsgas direkt in die Nase eines Benutzers, zum Beispiel zur Verwendung bei der Behandlung von Schlafstörungen, insbesondere bei Schlafapnoe.

Diese Vorrichtung (1) zum Zuführen von Atmungsgas direkt in die Nase eines Benutzers, umfasst:
- eine hohle und in einem Nasenloch des Benutzers einsetzbare elastische Membran (2); und
- ein im Hohlraum (5) der elastischen Membran (2) eingesetztes Teil (4), das einen Durchgang (6) und mindestens einen Ausatmungskanal (7) aufweist, welche (6,7) sich von einem Ende (8) bis zu einem gegenüberliegenden Ende (9) des eingesetzten Teils (4) erstrecken.

Die Erfindung betrifft auch ein Verfahren zur Herstellung der erfindungsgemässen Vorrichtung, ein Set und ein System zum Zuführen von Atmungsgas direkt in die Nase eines Benutzers sowie ein Verfahren zum Zusammenbauen des erfindungsgemässen Systems.

## Beschreibung

### Gebiet der Erfindung

Die Erfindung betrifft eine Vorrichtung zum Zuführen von Atmungsgas direkt in die Nase eines Benutzers, zum Beispiel zur Verwendung bei der Behandlung von Schlafstörungen, insbesondere bei Schlafapnoe.

### Hintergrund der Erfindung

Bei der Behandlung von Schlafstörungen, wie Schlafapnoe (das heisst, Atemstillstand während des Schlafens) werden üblicherweise Gesichtsmasken oder Nasenmasken eingesetzt, wie dies beispielweise aus der europäischen Patentanmeldung EP 1 314 445 bekannt ist.

Eine solche Maske deckt die ganze Nase und einen Teil des Gesichtes ab. Sie wird im Allgemeinen durch eine aus Bändern zusammengesetzte Halterung über dem ganzen Kopf festgehalten. Die Bänder ziehen die Maske mit ausreichender Kraft gegen das Gesicht, damit zwischen der Maske und dem Gesicht des Trägers eine gasdichte Abdichtung erreicht wird.

Bei der Behandlung eines Schlafapnoe-Syndroms muss der Patient eine solche Maske jede Nacht und während einer sehr langen Zeit, meist lebenslänglich, tragen. Eine Überdruckbeatmung wird mit der Maske durchgeführt. Diese Therapie ist unter dem Fachausdruck CPAP-Therapie bekannt. CPAP steht für "Continuous Positive Airway Pressure", d.h. Luftwege-Dauerüberdruck. Ein CPAP leitet einen von einem Ventilator erzeugten kontinuierlichen Luftstrom in die Maske, die der Patient während der ganzen Nacht tragen muss. Dadurch entsteht im Nasen-Rachenraum ein erhöhter Druck, der einen Kollaps der Atemwege verhindert. Der Patient kann normal atmen und die typischen Syndrome treten nicht mehr auf.

Ein wichtiger Nachteil der Anwendung von einer Maske besteht darin, dass die Maske mit einer verhältnismassig grossen Kraft gegen das Gesicht angebracht wird. Dadurch treten häufig Druckstellen im Gesicht, Gesichtwundheit und Gesichtvereiterung auf. Wenn die Bänder jedoch nicht genug angezogen sind, können Augenreizungen auftretten.

Ausserdem müssen für die hygienische Pflege und Reinigung der Maske und CPAP-Gerät täglich mindestens 15 bis 20 Minuten aufgewendet werden.

Ferner ist das Maskensystem ein Verbrauchsprodukt. Heutzutage benötigt ein Patient pro Jahr 2 bis 4 Maskensysteme, was zu einem grossen finanziellen und materiellen Aufwand führt.

Deshalb muss der Leidensdruck eines Schlafapnoe-Patienten sehr hoch sein, damit er den heutigen Stand der Technik der CPAP-Therapie akzeptiert und die Unannehmlichkeiten, Einschränkungen und Behinderungen durch den regelmässigen Gebrauch der Maske über eine Lange Zeit erträgt.

Die Erfindung bezweckt daher die Vermeidung oder Milderung der obigen Nachteile und Schwierigkeiten und stellt die Aufgabe, ein Hilfmittel zu schaffen, welches praktisch, leicht, komfortabel, einfach herzustellen und zu benutzen und preisgünstig ist und welches alle Kopf- und Körperbewegungen des Trägers im Schlaf mitmacht (störungsfrei begleitet).

### Offenbarung der Erfindung

Erfindungsgemäss wird diese Aufgabe durch eine Vorrichtung gelöst, welche
- eine hohle und in einem Nasenloch des Benutzers einsetzbare elastische Membran; und
- ein im Hohlraum der elastischen Membran einsetzbares Teil, das einen Durchgang und mindestens einen Ausatmungskanal aufweist, welche sich von einem Ende bis zu einem gegenüberliegenden Ende des eingesetzten Teiles erstrecken, umfasst.

Nach einer Ausführungsart der Erfindung weist die Membran mindestens ein abgerundetes Ende auf.

Nach einer weiteren Ausführungsart der Erfindung weist das eingesetzte Teil mindestens ein abgerundetes oder abgefastes Ende auf.

Nach einer weiteren Ausführungsart weist das eingesetzte Teil mindestens ein Querloch, welches sich in der Wandung des eingesetzten Teil zwischen dem Durchgang und der Aussenfläche des eingesetzten Teils erstreckt auf.

Gegenstand der Erfindung ist auch ein Verfahren zur Herstellung einer erfindungsgemässen Vorrichtung, bei dem ein Teil, welches einen Durchgang und mindestens einen Ausatmungskanal aufweist, welche sich von einem Ende bis zu einem gegenüberliegenden Ende des eingesetzten Teils erstrecken, in den Hohlraum einer hohlen und in einem Nasenloch des Benutzers einsetzbaren elastischen Membran eingefügt und die Membran an diesem Teil befestigt wird.

Ein weiterer Gegenstand der Erfindung ist ein Set zum Zuführen von Atmungsgas direkt in die Nase eines Benutzers, umfassend:
- eine erfindungsgemässe Vorrichtung; und
- eine Halterungseinrichtung zum Festhalten der erfindungsgemässen Vorrichtung in einem Nasenloch des Benutzers.

Nach einer Ausführungsart der Erfindung ist die Halterungseinrichtung an einem Zuführschlauch befestigbar, welcher das Atmungsgas abgeben kann.

Nach einer weiteren Ausführungsart weist die Halterungseinrichtung ein Verbindungsteil und ein Berührungsteil auf.

In vorteilhafter Weise weist das Verbindungsteil an einem Ende eine Bohrung für den Zuführschlauch auf und das Berührungsteil befindet sich an einem gegenüberliegenden Ende des Verbindungsteils.

Vorteilhaft hat das Berührungsteil im Wesentlichen die Form einer Platte, welche der Achse der Bohrung zugewandt ist. Diese Platte kann gegebenenfalls bogenförmig sein.

Eine weitere vorteilhafte Ausgestaltung der Erfindung besteht darin, dass die Fläche des Berührungsteils, die sich auf der Seite des Verbindungsteils befindet, mit einem Polster versehen ist.

Gegenstand der Erfindung ist auch ein System zum Zuführen von Atmungsgas direkt in die Nase eines Benutzers, umfassend:
- ein erfindungsgemässes Set und
- einen Zuführschlauch,
wobei der Zuführschlauch die Bohrung des Verbindungsteils der Halterungseinrichtung durchdringt und in mindestens einen Teil des Durchgangs des eingesetzten Teils der Vorrichtung des Sets eindringt.

Nach einer weiteren Ausführungsart weist der Zuführschlauch ein seitliches Loch auf, das Polster des Berührungsteils der Halterungseinrichtung ist aufblasbar und das Verbindungsteil weist einen Kanal auf, der sich von der Bohrung des Verbindungsteils bis zum Berührungsteils erstreckt, wobei das seitliche Loch des Zuführschlauchs dem Kanal zugewandt ist, um das Atmungsgas vom Schlauch bis zum aufblasbaren Polster zu führen.

Vorteilhaft sind auf dem Verbindungsteil Mittel zum Reduzieren des Zugangs des Atmungsgases zum Kanal vorgesehen.

Ein weiterer Gegenstand der Erfindung ist ein Verfahren zum Zusammenbauen eines erfindungsgemässen Systems, bei dem
- ein Zuführschlauch durch die Bohrung des Verbindungsteils einer Halterungseinrichtung geführt und in mindestens einen Teil des Durchganges des eingesetzen Teils der erfindungsgemässen Vorrichtung eingefügt wird; und
- gegebenenfalls der Abstand zwischen dem Verbindungsteil und der Vorrichtung eingestellt wird.

### Kurze Beschreibung der Zeichnungen

Im Folgenden werden Ausführungsformen der Erfindung unter Bezugnahme auf die begleitenden Zeichnungen beschrieben. Es zeigen :
Figur 1 eine perpesktivische Darstellung einer erfindungsgemässen Vorrichtung;
Figur 2 eine Ansicht einer weiteren Ausführungsform der erfindungsgemässen Vorrichtung;
Figur 3 eine perspektivische Darstellung einer Halterungseinrichtung zum Festhalten der erfindungsgemässen Vorrichtung in einem Nasenloch des Benutzers;
Figur 4 eine perspektivische Darstellung eines erfindungsgemässen Systems;
Figur 5 eine perspektivische Darstellung eines weiteren erfindungsgemässen Systems mit einem bogenförmigen Verbindungsteil; und
Figur 6 einen Teil des Kopfs eines Benutzers bei der Verwendung des erfindungsgemässen Systems.

### Beschreibung der Ausführungsformen der Erfindung

### Erfindungsgemässe Vorrichtung

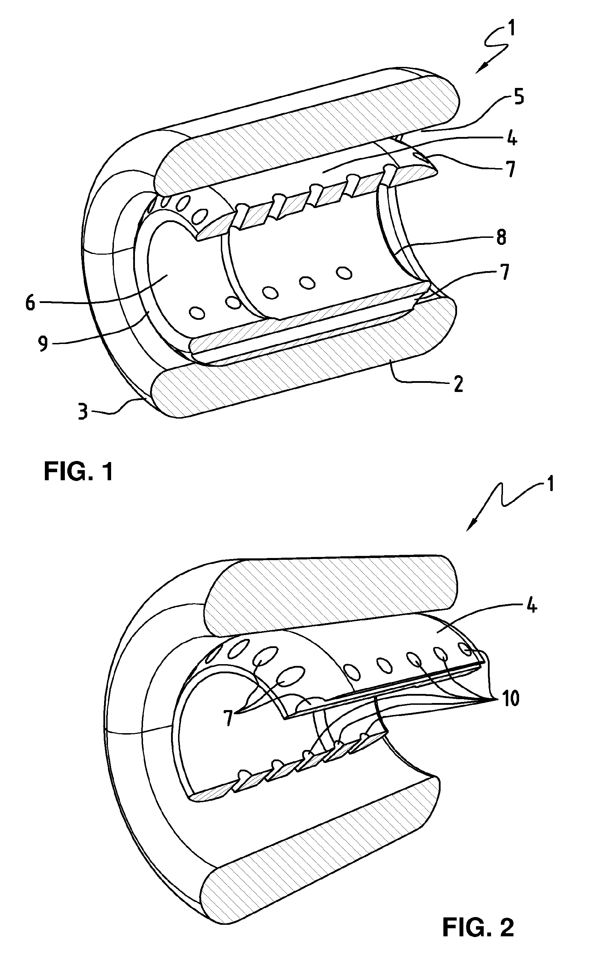

Die erfindungsgemässe Vorrichtung 1 wird in Fig. 1 dargestellt. Sie umfasst eine hohle Membran 2, die aus einem elastischen Material besteht. "Elastisch" heisst hier, dass sich die Membran an der Form des Nasenlochs anpassen können muss. Das Material kann ein natürliches Material wie zum Beispiel Kautschuk sein oder ein künstliches Material wie zum Beispiel ein Elastomer. Es muss für einen Kontakt mit der Nase geeignet sein. Hierzu muss berücksichtig werden, das die Membran eine ganze Nacht bzw. mehrere Stunden (im Allgemeinen 2 bis 12 Stunden) im Nasenloch des Benutzers bleiben wird. Als Material kann insbesondere Silikonkautschuk verwendet werden.

Die äusseren Abmessungen der Membran sind so ausgewählt, dass die Membran in einem Nasenloch des Benutzters einfach eingesetzt werden kann.

Vorzugsweise ist mindestens das Ende 3 der Membran, das in die Nase eingeführt werden muss, abgerundet.

Die erfindungsgemässe Vorrichtung 1 weist ferner ein Teil 4 auf, das im Hohlraum 5 der Membran 2 eingesetzt ist. Dieses Teil 4 weist einen Durchgang 6 auf. In der Wandung des eingesetzten Teils 4 befindet sich mindestens ein Ausatmungskanal 7. Sowohl der Durchgang 6 als auch der Atmungskanal 7 erstrecken sich von einem Ende 8 bis zu einem gegenüberliegenden Ende 9 des eingesetzten Teils 4.

Der Durchgang 6 ermöglicht dem Benutzter das Zuführen von Atmungsgas und der Ausatmungskanal oder die Ausatmungskanäle 7 dienen zur Ausatmung des Benutzters.

Das eingesetzte Teil 4 besteht im Allgemeinen aus einem steifen Material, das keine Nasenreizungen verursachen kann. Als solches Material können insbesondere Kunststoffe eingesetzt werden.

Vorzugsweise ist mindestens ein Ende 8 oder 9 des Teils 4 abgefast oder abgerundet, um sein Einfügen in den Hohlraum 5 der Membran 2 zu erleichtern.

Die Membran 2 und das eingesetzte Teil 4 sind im Allgemeinen zylinderförmig, da diese Form einfach zu erhalten bzw. herzustellen ist. Der Durchgang 6 und der Atmungskanal 7 verlaufen im Allgemeinen parallel zur gemeinsamen Achse der Membran 2 und des Teils 4, d.h., vom axialen Ende 8 bis zum anderen axialen Ende 9. Die axiale Länge des eingesetzten Teils 4 ist im Allgemeinen kleiner als die axiale Länge der Membran 2.

Die Membran 2 kann beispielsweise einen Durchmesser von 5 bis 8 mm und eine axiale Länge von 10 bis 15 mm haben, während das Teil 4 einen Durchmesser von 3 bis 6 mm und eine axiale Länge von 8 bis 13 mm haben kann. Der Durchmesser des oder der Atmungskanäle 7 beträgt zum Beispiel 1 mm.

Der Querschnitt der Membrane 2 und des eingesetzten Teils 4 könnte auch oval sein.

Vorzugsweise hat das eingesetzte Teil 4 mehrere Ausatmungskanäle 7, zum Beispiel acht.

Nach einer Ausführungsart, die in Fig. 2 dargestellt ist, weist das eingesetzte Teil 4 mindestens ein Querloch 10 auf, das sich in der Wandung des eingesetzten Teils 4 zwischen dem Durchgang 6 und der Aussenfläche erstreckt. Dieses Querloch 10 dient zum Erzeugen von einem Druck gegen die Membrane 2, was vorteilhaft zu einer Positionierung und Fixierung der Vorrichtung 1 in dem Nasenloch des Benuzters führt. Der Durchmesser dieses Querloches 10 beträgt zum Beispiel 1 mm.

Vorzugsweise sind mehrere Querlöcher 10, zum Beispiel zwölf, vorhanden. Vorteilhafterweise sind die Öffnungen dieser Querlöcher 10 auf der Aussenfläche des eingesetzten Teils 4 so angeordnet, dass ein Druck auf der Innerfläche der Membrane 2 in allen radialen Richtungen ausgeübt wird.

### Herstellung der erfindungsgemässen Vorrichtung

Um die Vorrichtung 1 herzustellen, wird das Teil 4 in die Membrane 2 eingefügt. Die Membrane 2 wird dann am Teil 4 befestigt. Diese Befestigung kann chemisch (z.B. durch Vulkanisation), thermisch (z.B. durch Laserstrahlung oder Ultraschallverschweissung) oder durch irgendwelche geeignete Methode erfolgen.

Vorzugsweise werden nur die Ende der Membran 2 luftdicht an den Enden des Teils 4 befestigt, damit sich die Membran 2 wegen des Druckes des Atmungsgases zwischen ihren befestigten Enden vom Teil 4 entfernt und sich somit optimal an der Form des Nasenloches anpasst und an der Nase des Benutzers festgehalten wird.

### Erfindungsgemässes Set

Das erfindungsgemässe Set umfasst eine erfindungsgemässe Vorrichtung und eine Halterungseinrichtung zum Festhalten der Vorrichtung in einem Nasenloch des Benutzers.

Diese Halterungseinrichtung ist vorteilhafterweise vorgesehen, um an einem Zuführschlauch, der das Atmunsgas in die Nase liefert, befestigt zu werden.

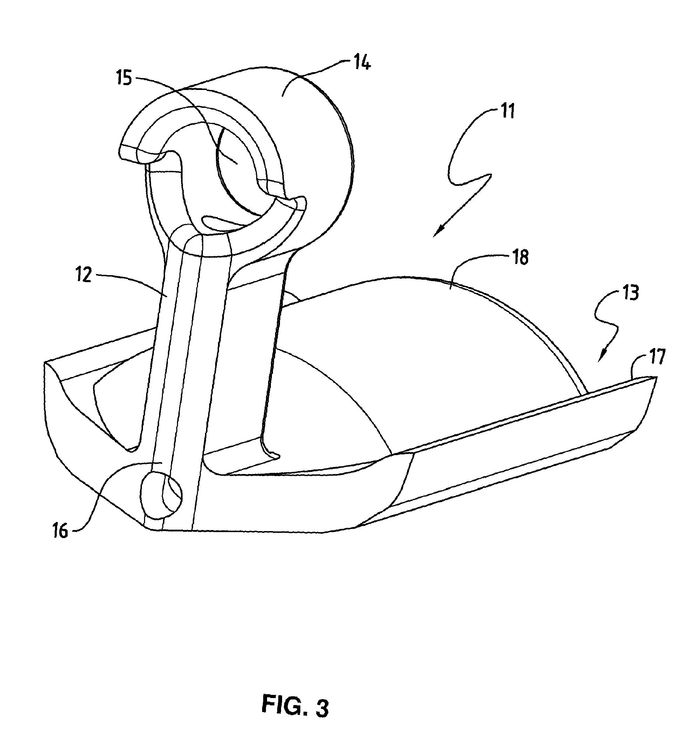

In Fig. 3 ist eine solche Halterungseinrichtung 11 dargestellt. Sie weist ein Verbindungsteil 12 und ein Berührungsteil 13 auf.

Das Verbindungsteil 12 hat eine im Wesentlichen längliche Form. An einem Ende 14 weist es eine Bohrung 15 auf, die zur Lagerung des Zuführschlauchs geeignet ist. Die Achse der Bohrung 15 ist im Wesentlichen senkrecht zur Längsachse des Verbindungsteils 12.

An einem gegenüberliegenden Ende 16 des Verbindungsteils 12 befindet sich das Berührungsteil 13. Dieses hat im Wesentlichen die Form einer Platte, die der Achse der Bohrung zugewandt ist, d.h. dass die Ebene, die durch die Hauptflächen der Platte gebildet wird, einen im Wesentlichen rechten Winkel mit der Längsachse des Verbindungsteil 12 bildet.

Die Halterungseinrichtung 11, insbesondere das Verbindungsteil 13, besteht im Allgemeinen aus einem Kunstoff, der für einen Kontakt mit der Nase geeignet ist.

Vorteilhafterweise ist die Fläche 17 des Berührungsteils, die sich auf der Seite der Bohrung 15 befindet, mindestens teilweise mit einem Polster 18 versehen. Dieses Polster 18 kann aus Materialen wie z.B. Polytetrafluoroethylen (PTFE) oder expandiertes PTFE bestehen. Es kann steif oder weich sein.

Der Abstand zwischen dem Berührungsteils 13 oder gegebenfalls dem Polster 18 und der Membran 2 entspricht normalerweiser der Dicke eines Nasenflügels, d.h. 2 bis 3 mm.

### Erfindungsgemässes System und sein Zusammenbauen

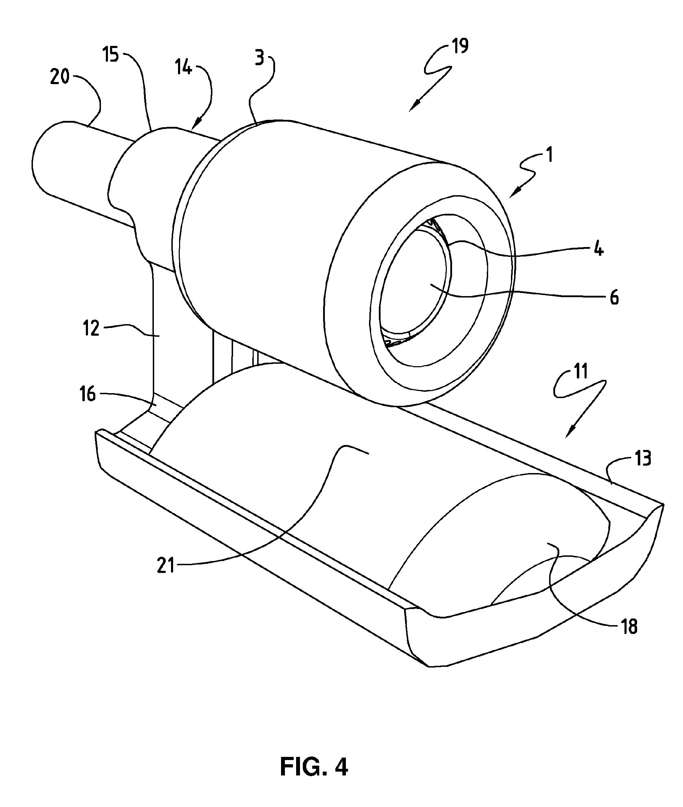

Das erfindungsgemässe System 19 ist in Fig. 4 dargestellt. Es umfasst ein erfindungsgemässes Set und einen Zuführschlauch 20, der zur Lieferung von Atmungsgas in der Nase vorgesehen ist.

Der Zuführschlauch 20 dringt durch die Bohrung 15 der Halterungseinrichtung 11 und mindestens in einen Teil des Durchgangs 6 des eingesetzen Teils 4. Die Achse der Vorrichtung 1 ist also parallel zu der durch die Hauptflächen des Berührungsteils 13 gebildeten Ebene.

Ein Zwischenraum 21 ist durch die Vorrichtung 1 und das Behrührungsteil 13 und das eventuelle Polster 18 gebildet.

Um dieses Systems zu montieren, kann zum Beispiel zuerst der Zuführschlauch 20 durch die Bohrung 13 eingeführt und dann in mindestens einen Teil des Durchgangs 6 des eingesetzten Teils 4 der Vorrichtung 1 eingefügt werden. Für den Fall, dass das Teil 4 Querlöcher 10 aufweist, wird es selbstverständlich vermieden, den Zuführschlauch 20 in den ganzen Durchgang 6 einzuführen, sonst werden diese Querlöcher 10 verstopft.

Der Durchmesser der Bohrung 15 ist so gewählt, dass sich ein kleiner Spielraum zwischen der Bohrung 15 und dem Zuführungschlauch 20 ergibt.

Der Durchmesser des Durchgangs 6 ist so gewählt, dass es keinen Spielraum zwischen dem Zuführschlauch 20 und dem Durchgang 6 gibt, damit der Zuführschlauch 20 fest im Durchgang 6 gehalten wird.

Es kann ein axialer Abstand zwischen dem Ende 14 des Verbindungsteils 12 und dem Ende 3 der Vorrichtung 1 bestehen.

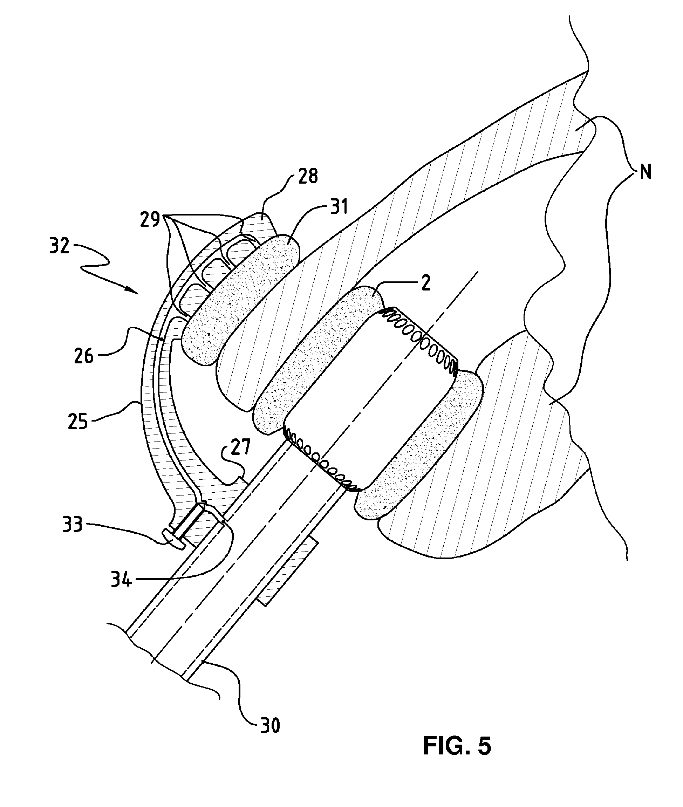

In Fig. 5 ist eine weitere Ausführungsart des erfindungsgemässen Systems dargestellt.

Das Verbindungsteil 25 der Halterungseinrichtung 32 weist hier einen Kanal 26 auf, der sich von der Bohrung 27 des Verbindungsteils 25 bis zum Berührungsteils 28 erstreckt. Am Berührungsteil 28 endet der Kanal 26 mit mindestens einer Düse 29, die in ein aufblasbares Polster 31 mündet.

Der Zuführschlauch 30 weist zusätzlich zur seiner normalen Öffnung ein seitliches Loch 34 auf, das dem Kanal 26 zugewandt ist, um das Atmungsgas vom Zuführschlauch 30 bis zum aufblasbaren Polster 31 zu führen.

Auf diese Weise kann das Atmungsgas durch das seitliche Loch 34, den Kanal 26 und die Düse 29 fliessen und das Polster 31 aufblasen. Dadurch wird der Nasenflügel zwischen dem Polster 31 und der Membran 2 gepresst und das erfindungsgemässe System an der Nase N des Benutzers befestigt.

Beim Montieren des Systems der Figur 5 wird gegebenenfalls der Zuführschlauch 30 gedreht, bis sein seitliches Loch 34 in den Kanal 26 mündet.

Vorzugsweise werden auch Mittel 33 wie ein Ventil auf dem Verbindungsteil 25 auf der Seite der Bohrung 27 vorgesehen, welche Mittel 33 den Zugang des Atmungsgases zum Kanal 26 reduzieren, um den Druck des Polsters 31 auf dem Nasenflügel des Benutzers einzustellen bzw. zu vermindern.

### Verwendung des erfindungsgemässen Systems

Ein Schlafapnoe-Syndrom wird heutzutage ausschliesslich durch einen Pneumologen diagnostiziert, der dann die angemessene Therapie verschreibt. Die inviduelle Einstellungs des CPAP-Gerätes und die Anpassung und Kontrolle des erfindungsgemässen Systems können in einem Schlaflabor erfolgen.

Die Abmessungen der erfindungsgemässen Vorrichtung und Halterungseinrichtung, sowie der axiale Abstand zwischen der Vorrichtung und dem Verbindungsteil der Halterungseinrichtung werden je nach Benuzter bzw. Patienten bestimmt bzw. eingestellt.

Die erfindunsgemässe Vorrichtung und gegebenenfalls die dazugehörige Halterungseinrichtung sind vorzugsweise für einen einmaligen Gebrauch bestimmt und werden nach der Anwendung entsorgt.

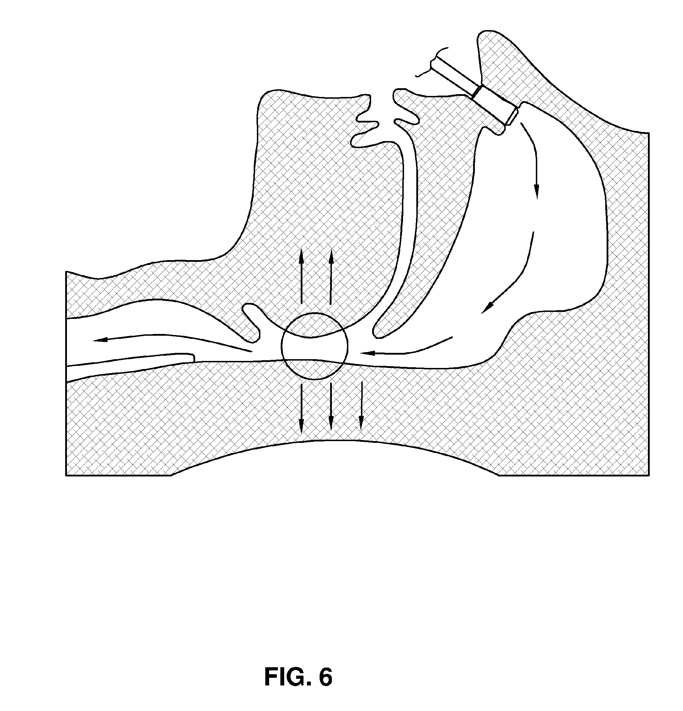

Wie aus der Fig. 6 ersichtlich, steckt sich der Benutzer bzw. Patient bei der Verwendung die erfindungsgemässe Vorrichtung in ein Nasenloch. Der entsprechende Naseflügel wird dann durch die Zusammenwirkung der Vorrichtung und der (nicht dargestellten) Halterungseinrichtung geklemmt, wodurch das System festgehalten wird. Diese Befestigung hält allen Kopf- und Körperbewegungen während des Schlafs des Benutzers stand. Es werden zwei erfindungsgemässe Systeme gleichzeitig gebraucht. Der Zuführschlauch 20 oder 30 kann dann mit dem Druckluftschlauch eines Ventilators (oder einer Pumpe) verbunden werden. Gegebenenfalls stellt der Benutzer den Fluss des Atmungsgases bzw. den Druck auf seinem Nasenflügel ein, indem er auf die Mittel 33 (siehe Fig. 5) einwirkt.

Es kann auch passieren, dass der Benutzer keine Halterungseinrichtungen benötigt und die Vorrichtungen alleine anwendet.

Mit dem erfindungsgemässen System wird die von einem CPAP-Gerät mit leicht erhöhtem Überdruck erzeugte Atemluft direkt in die Nase des Benutzers bzw. Patienten zugeführt. Dadurch wird erstmals eine Therapierung der Schlafapnoe ohne unangenehme Gesichtsmaske und ohne störende Kopfbänder möglich.

In dieser Weise werden die Beeinträchtigungen des Benutzers bzw. Patienten wesentlich reduziert, die Anwendung der unterstützten Beatmung vereinfacht und die Akzeptanz der Therapie erheblich verbessert.

## Patentansprüche

1. Vorrichtung (1) zum Zuführen von Atmungsgas direkt in die Nase eines Benutzers, umfassend:
- eine hohle und in einem Nasenloch des Benutzers einsetzbare elastische Membran (2); und
- ein im Hohlraum (5) der elastischen Membran (2) eingesetztes Teil (4), das einen Durchgang (6) und mindestens einen Ausatmungskanal (7) aufweist, welche (6,7) sich von einem Ende (8) bis zu einem gegenüberliegenden Ende (9) des eingesetzten Teils (4) erstrecken.

2. Vorrichtung (1) nach Anspruch 1, **dadurch gekennzeichnet, dass** die Membran (2) mindestens ein abgerundetes Ende (3) aufweist.

3. Vorrichtung (1) nach nach Anspruch 1 oder 2, **dadurch gekennzeichnet, dass** das eingesetzte Teil (4) mindestens ein abgerundetes oder abgefastes Ende (8,9) aufweist.

4. Vorrichtung (1) nach Anspruch 1, **dadurch gekennzeichnet, dass** die Membran (2) und das eingesetzte Teil (4) im Wesentlichen zylinderförmig sind.

5. Vorrichtung (1) nach einem der Ansprüche 1 bis 4, **dadurch gekennzeichnet, dass** das eingesetzte Teil mindestens ein Querloch (10) aufweist, welches sich in der Wandung des eingesetzten Teils (4) zwischen dem Durchgang (6) und der Aussenfläche des eingesetzten Teils (4) erstreckt.

6. Vorrichtung (1) nach einem der Ansprüche 1 bis 5, **dadurch gekennzeichnet, dass** die Membran (2) aus Kautschuk, insbesondere Silikonkautschuk besteht.

7. Vorrichtung (1) nach einem der Ansprüche 1 bis 6, **dadurch gekennzeichnet, dass** das eingesetzte Teil (4) aus Kunstoff besteht.

8. Vorrichtung (1) nach einem der Ansprüche 1 bis 7, **dadurch gekennzeichnet, dass** die Halterungseinrichtung (11) aus Kunstoff besteht.

9. Verfahren zur Herstellung einer Vorrichtung (1) nach einem der Ansprüche 1 bis 8, bei dem ein Teil (4), welches einen Durchgang (6) und mindestens einen Ausatmungskanal (7) aufweist, welche (6,7) sich von einem Ende (8) bis zu einem gegenüberliegenden Ende (9) des eingesetzten Teils (4) erstrecken, in den Hohlraum (5) einer hohlen und in einem Nasenloch des Benutzers einsetzbaren elastischen Membran (2) eingefügt und die Membran (2) an diesem Teil (4) befestigt wird.

10. Set zum Zuführen von Atmungsgas direkt in die Nase eines Benutzers, umfassend:
- eine Vorrichtung (1) gemäss einem der Ansprüche 1 bis 8 oder eine Vorrichtung hergestellt durch das Verfahren gemäss Anspruch 9; und
- eine Halterungseinrichtung (11,32) zum Festhalten der besagten Vorrichtung (1) in einem Nasenloch des Benutzers.

11. Set nach Anspruch 10, **dadurch gekennzeichnet, dass** die Halterungseinrichtung (11,32) an einem Zuführschlauch (20,30), welcher das Atmungsgas abgeben kann, befestigbar ist.

12. Set nach Anspruch 11, **dadurch gekennzeichnet, dass** die Halterungseinrichtung (11,32) ein Verbindungsteil (12,25) und ein Berührungsteil (13,28) aufweist.

13. Set nach Anspruch 12, **dadurch gekennzeichnet, dass** das Verbindungsteil (12,25) eine Bohrung (15,27) für den Zuführschlauch (20,30) an einem Ende (14) aufweist und dass das Berührungsteil (13,28) sich an einem gegenüberliegenden Ende (16) des Verbindungsteils (12,25) befindet.

14. Set nach Anspruch 13, **dadurch gekennzeichnet, dass** das Berührungsteil (13,28) im Wesentlichen die Form einer Platte hat, welche der Achse der Bohrung (15,27) zugewandt ist.

15. Set nach Anspruch 14, **dadurch gekennzeichnet, dass** die Fläche (17) des Berührungsteils (13,28), die sich auf der Seite des Verbindungsteils (12,25) befindet, mit einem Polster (18,31) versehen ist.

16. System zum Zuführen von Atmungsgas direkt in die Nase eines Benutzers, umfassend:
- ein Set gemäss einem der Ansprüche 10 bis 15 und
- einen Zuführschlauch (20,30),
wobei der Zuführschlauch (20,30) die Bohrung (15,27) des Verbindungsteils (12,25) der Halterungseinrichtung (11,32) durchdringt und in mindestens einen Teil des Durchgangs (6) des eingesetzten Teils (4) der Vorrichtung (1) des besagten Sets eindringt.

17. System nach Anspruch 16, **dadurch gekennzeichnet, dass** der Zuführschlauch (30) ein seitliches Loch (34) aufweist, das Polster (31) des Berührungsteils (28) der Halterungseinrichtung (32) aufblasbar ist und das Verbindungsteil (25) einen Kanal (26) aufweist, der sich von der Bohrung (27) des Verbindungsteils (25) bis zum Berührungsteils (28) erstreckt, wobei das seitliche Loch (34) des Zuführschlauchs (30) dem Kanal (26) zugewandt ist.

18. System nach Anspruch 17, **dadurch gekennzeichnet, dass** Mittel (33) zum Reduzieren des Zugangs des Atmungsgases zum Kanal (26) auf dem Verbindungsteil (25) vorgesehen sind.

19. Verfahren zum Zusammenbauen eines Systems nach einem der Ansprüche 16 bis 18, **dadurch gekennzeichnet, dass**
ein Zuführschlauch (20,30) durch die Bohrung (15,27) des Verbindungsteils (12,25) einer Halterungseinrichtung (11,32) geführt und in mindestens einen Teil des Durchganges (6) des eingesetzen Teils (4) einer Vorrichtung (1) gemäss einem der Ansprüche 1 bis 8 oder einer Vorrichtung hergestellt durch das Verfahren gemäss Anspruch 9 eingefügt wird; und
gegebenenfalls der Abstand zwischen dem besagten Verbindungsteil (12,25) und der besagten Vorrichtung (1) eingestellt wird.

20. Verfahren nach Anspruch 19, **dadurch gekennzeichnet, dass** der Zuführschlauch (30) gedreht wird, bis sein seitliches Loch (34) in den Kanal (26) des Verbindungsteils (25) mündet.
